# EUROPEAN PATENT SPECIFICATION

(11) **EP 0 571 337 B1**
(45) Date of publication and mention of the grant of the patent: **30.06.1999**
(21) Application number: 93830191.8
(22) Date of filing: 03.05.1993
(51) Int. Cl.: C07K 1/20, C07K 1/30, C07K 1/36

(54) **Process to purify proteins from cell systems**
Verfahren zur Reinigung von Proteinen aus Zellsystemen
Procédé de purification de protéins à partir de systèmes cellulaires

(30) Priority: 22.05.1992 IT RM920385
(43) Date of publication of application: 24.11.1993
(73) Proprietor: WABCO B.V., 7940 KB Meppel (NL)
(72) Inventor: Bonelli, Fabrizio, I-15033 Casale Monferrato, Alessandria (IT); Cecconato, Elvi, I-10034 Chivasso, Torino (IT); Calogero, Raffaele, I-80038 Pomigliano d'Arco, Napoli (IT)
(74) Representative: de Simone, Domenico

(56) References cited:
- EP-A- 0 480 048
- METHODS IN ENZYMOLOGY vol. 182, no. 1, 1990, ACADEMIC PRESS, INC. NEW YORK, US;, pages 285 - 300 M.P. DEUTSCHER (ED.) 'Guide to protein purification'
- DATABASE WPI Week 9003, Derwent Publications Ltd., London, GB; AN 90-020000 & JP-A-1 300 898 (AJINIMOTO KK) 05 December 1989 'Purification of Physiologically Active Protein'
- BIO/TECHNOLOGY vol. 7, no. 7 , July 1989 , NATURE AMERICA, INC., NEW YORK, US; pages 690 - 697 A. MITRAKI AND J. KING 'Protein folding intermediates and inclusion body formation'
- BIO/TECHNOLOGY vol. 8, no. 10 , October 1990 , NATURE AMERICA, INC., NEW YORK, US; pages 945 - 949 P.C. BABBITT ET AL. 'Removal of a proteolytic activity associated with aggregates formed from expression of creatine kinase in Escherichia coli leads to improved recovery of active enzyme'

## Description

This invention relates to a process to purify proteins, more generally polypeptides, with a basic isoelectric point from both prokaryotic and eukaryotic heterologous cell systems.

Recombinant proteins, obtained by introduction and expression of nucleic acids in heterologous cell systems, allow to produce polypeptides on an industrial scale basis. Either the expression level and the functional activity of the recombinant protein are largely affected by the chosen cell system. In prokaryotic systems, particularly *Escherichia coli,* the protein activity is often either diminished or inhibited by the occurrence of insoluble aggregate occurrence (1-4), known as "inclusion bodies". In the inclusion body forming process, interchain interactions between the newly synthesised recombinant protein and host proteins, rather than physiological intrachain interactions, are promoted, both by the reducing environment of *E. coli* cytoplasm and by the lack of cell compartments into bacterial cells. Furthermore, proteins of the bacterial plasmatic membrane have been found to associate with the expressed recombinant protein (5), and are therefore involved in forming such inclusion bodies.

Moreover, either the protein expression level and the immunological and enzymatic activity may result to be affected by host co-purifying protease activities (6,7), and cause a significant lowering of the process yield. This occurs on both prokaryotic and eukaryotic expression systems.

Further, as noted by the authors of the present invention, the transport into nuclei of nucleic acid binding recombinant proteins (8-11), or the interaction thereof with cytoplasm nucleic acids, in eukaryotic systems, make difficult to establish a standard purification procedure. Although the nucleic acid-protein interaction could be destroyed by using SDS (sodium dodecylsulphate), this prevents the use of both ion exchange and reverse phase chromatographies, very advantageous and largely used as purification techniques.

On the other side, use of DNAse or RNAse does not succeed in fully removing the nucleic acids. Furthermore, oligonucleotides deriving by the enzymatic digestion of nucleic acids, are still able to react with proteins having a basic isoelectric point and to form nucleic acid-protein aggregates. Such aggregates have very large variability chromatography behaviours and thus negatively affect the repeatability of the purifying process on different batches.

A scope of this invention is to solve the purification and poor stability protein troubles of intracellular proteins having a basic isoelectric point, both from prokaryotic or eukaryotic systems.

A further scope of this invention is to supply a protein purification method, which allows to obtain highly purified and homogeneous proteins, stabilised against thermal stresses (such as frequent freezing/thawing procedures) and showing functional and/or immunologic activities similar to polypeptides from natural sources.

The authors found that, treatment of protein expressing cells, both from prokaryotic or eukaryotic systems, or a membrane fraction thereof, with a denaturing agent, such as the guanidine hydrochloride (CH₆ClN₃), in an acid environment, followed by precipitation with an organic solvent, such as acetonitrile (CH₃CN), causes the breaking of the interactions between the nucleic acids and the recombinant protein; a solubilization of aggregates between recombinant and membrane proteins ("inclusion bodies" in the case of *E. coli)* ; the inhibition of protease activities and the removal of nucleic acids and of most of cytoplasm and membrane proteins from the solution.

When used in the context of this invention, the term "basic protein" means a protein having an isoelectric point or a pH higher than 7.0; the term "acid protein " means a protein having an isoelectric point or a pH lower than 7.0.
- processing cells, or an already purified fraction thereof, containing said proteins with basic isoelectric point, with a denaturing agent under acid environment conditions, in order to obtain a mixture;
- precipitating said mixture by using an organic solvent so to obtain a pellet and a supernatant;
- fractionating said supernatant by chromatography.

According to this invention, said denaturing agent comprises a guanidine salt, more preferably guanidine hydrochloride, still more preferably at a concentration comprised between 6M and 8M, most preferably 7M.

Furthermore, according to this invention, said organic solvent comprises acetonitrile in the presence of a volatile acid; preferably said volatile acid is trifluoro acetic acid; more preferably the acetonitrile final concentration is in the range from 20 % to 30 % v/v, most preferably 25 % v/v; and the trifluoroacetic acid final concentration is most preferably in the range from 0.02 % and 0.25 % v/v.

According to a preferred embodiment of this invention, said phase chromatography comprises a chromatography on an inert resin, preferably silica having carbon aliphatic chains, preferably on a reverse phase, wherein said protein is eluted with said organic solvent.

In a preferred embodiment of this invention, said heterologous cell systems are prokaryotic cells, more preferably bacterial cells, most preferably E. *coli* cells.

Alternatively said heterologous cell systems are eukaryotic cells, preferably yeast, insect or mammalian cells, most preferably *Spodoptera frugiperda* cells.

According to a preferred embodiment of this invention, said protein having a basic isoelectric point is the recombinant delta antigen of Hepatitis D Virus (r-HDAg); alternatively, is the recombinant core antigen of Hepatitis C Virus (r-p22).

This invention will be described in the following explanatory, but not limiting, Examples, by making reference to the enclosed drawings, wherein:
- fig. 1 shows the step purification process according to this invention;
- fig. 2 shows a 12.5 % SDS-PAGE gel; the arrow indicates the r-HDAg corresponding band (31 kD); lane A: total proteins following an acetonitrile precipitation; MW: molecular weight standards (BioRad, USA); lane B: rHDAg after chromatography on C18; lane C: r-HDAg after chromatography on TSK SW 4000;
- fig. 3 shows a chromatography profile of the C18 elution product, wherein the outlined peak contains rHDAg;
- fig. 4 shows a RIA assay (AB-DELTAK (RIA), SORIN Biomedica, Italy) with HDV-antibodies, wherein samples from PP1 to PP6 are HDV immunoreactive; serums from SN7 to SN9 are HDV immunoreactivity free; values of immunoinhibition higher than 50 % are considered to be anti-HDV positive;
- fig. 5 shows a 15% SDS PAGE gel; the arrow indicates the r-p22 corresponding band (22 kD); lane 1: molecular weight standards (BioRad, USA); lane 2: total proteins after a CH₃CN (acetonitrile) precipitation; lane 3: r-p22 after a chromatography on C4;
- fig.6 shows a chromatography profile of the elution product from C4, wherein the outlined peak contains r-p22;
- fig.7 shows a panel of commercially available anti-HCV certified serums (1-25 BBI, USA), EIA assayed (ETI-AB-HCVK, SORIN Biomedica, Italy) with r-p22 protein (HCV core protein) as purified according to this invention when compared with a commercially available assay to detect HCV antibodies (ORTHO HCV ELISA Second Generation, Ortho Diagnostic Systems, USA). The 0 to 4 values on the histogram show the antiHCV core protein immunoreactivity of the serum panel, as defined by the CHIRON RIBA HCV Second Generation Control Test (CHIRON, USA).

### Example 1 Purification of Proteins From Heterologous Cell System Purification

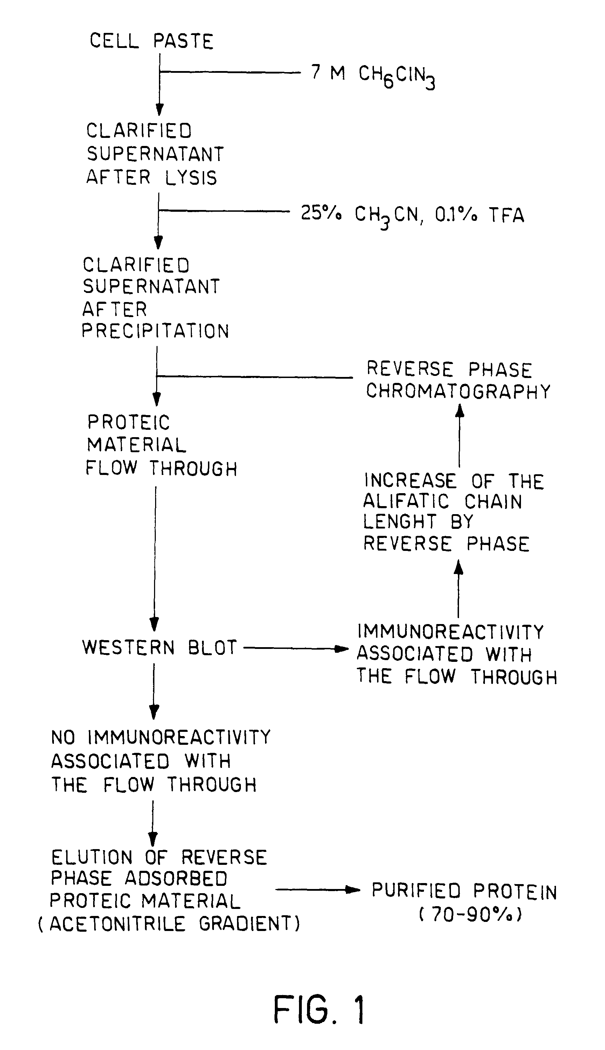

A scheme of the process is shown in fig.1.

### Cell Paste Lysis

The cell paste (10 g of bacterial cells, or 1 x 10⁹ eukaryotic cells) is suspended in 7M guanidine hydrochloride, 50 mM acetate buffer pH 5 (A buffer; 0.5 g/ml of cell paste or 1 x 10⁸ cells/ml). The suspension is submitted to 100 W ultrasonic impulses for one min, using a 10 nm tip, and the procedure repeated four times. Then the suspension is centrifuged at 100,000 x g for 30 minutes at 5°C. The supernatant shows a protein contents in the range from 15 up to 30 mg/ml.

### Organic Precipitation

The supernatant is 1:1 diluted with B solution, comprising: 50 % CH₃CN (acetonitrile) and 0.2 % TFA (trifluoroacetic acid) in water. The suspension is stirred for 60 minutes at 5°C and then centrifuged at 100,000 x g for 30 minutes. The supernatant from the centrifugation shows a protein contents 10-30 fold lower than the lysis protein contents (total proteins: 1-3 mg/ml).

### Reverse Phase Chromatography

The supernatant from the organic precipitation is loaded on C2 reverse phase silica column (19 mm x 300 mm, WATERS, Massachussets, USA). A fraction of the unadsorbed protein material is analysed on western blot , using conventional methods, for the immunoreactivity of the recombinant protein. The remaining unadsorbed protein material is loaded on a C4 reverse phase column (19 mm x 300 mm). A fraction of the unadsorbed protein material is analysed on western blot for the presence of the recombinant protein. Then the remaining material is treated on reverse phase columns with aliphatic chains of different length until conditions, under which the recombinant protein is adsorbed on the resin, are found. Then the protein is eluted by an acetonitrile gradient.

The protein that is obtained from this procedure shows a purity level in the range from 70 to 90 % (the purity level having been established following to a density measurement analysis of Coomassie Blue dyed SDS PAGE gels).

### Example 2 rHDAg From E. coli Purification Lysis

The cell paste from 500 ml of bacterial culture (2 g appr.) of W3110 (ATCC 27325), transformed with the plasmid pSORIN delta (DSM 6774, EP Application No. 0485347),is resuspended in 5 ml of A buffer.

The suspension is treated as in Example 1. The insoluble material from the centrifugation is then resuspended in 5 ml of the A buffer and centrifuged at 100,000 x g for 15 minutes at 10°C. The clarified supernatant from the lysis, as well as the supernatant from the second cell membrane, are then pooled (Pool-1).

### Organic Precipitation

The Pool-1 is 1:1 diluted with the B solution and incubated at 5°C under stirring for 60 minutes. Then the suspension is centrifuged at 100,000 x g for 15 minutes.

### Reverse Phase Chromatography (C18)

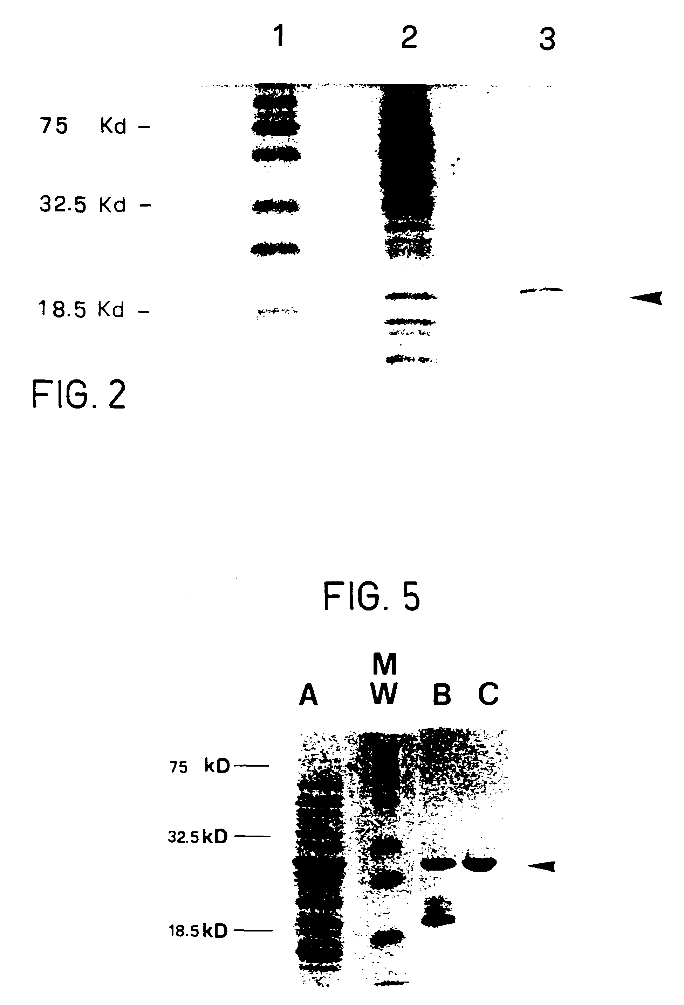

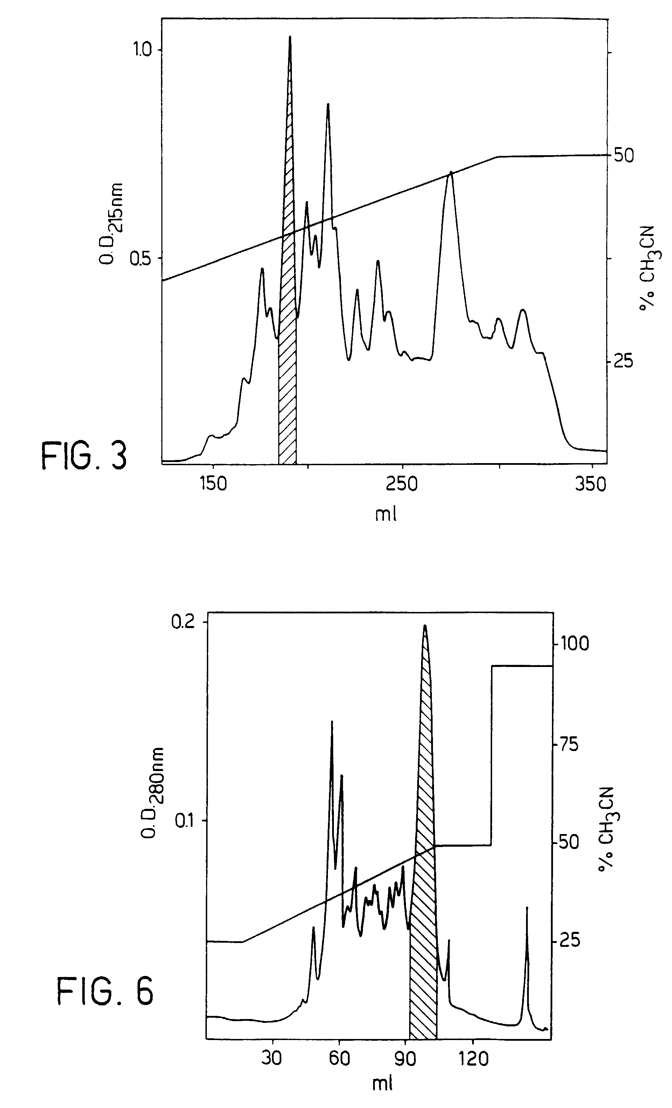

The supernatant from the organic precipitation (A, fig.2) is loaded on a C18 column (1.9 cm x 30 cm, 300 A Deltapac-Waters), balanced with 25 % CH₃CN in 0.1 % TFA (flow-rate: 10 ml/min; detector: 215 nm). The column is washed with 25 % CH₃CN and 0.1 % TFA until the base line reached the zero (O). Then the column is eluted with a 25-30 % CH₃CN gradient in a 0.1 % TFA gradient (gradient time: 30 minutes; flow rate: 10 ml/min.; detector: 215 nm). The outlined peak on the curve of the fig.3 contains the recombinant r-HDAg at a 60 % purity level (B in fig.2).

### Gel Permeation

The r-HDAg from the reverse phase chromatography is lyophilised and resuspended in the B solution, with 500 µl (0.5 %) of β-mercaptoethanol. The r-HDAg is loaded on TSK SW4000 column (7.8 mm x 600 mm, Waters, Massachussets, USA), balanced with the B solution (flow rate: 1 ml/min.; detector: 215 nm). The elution products are collected as 250 µl fractions. These fractions, after having been lyophilised, are dissolved in 2 % acetic acid and analysed on 12.5% PAGE SDS. The fractions containing r-HDAg (31 kD) are pooled (Pool-2; C in fig.2); the protein shows a purity level higher than 90 %.

### r-HDAg And Natural Antigen Immunoreactivity Comparison

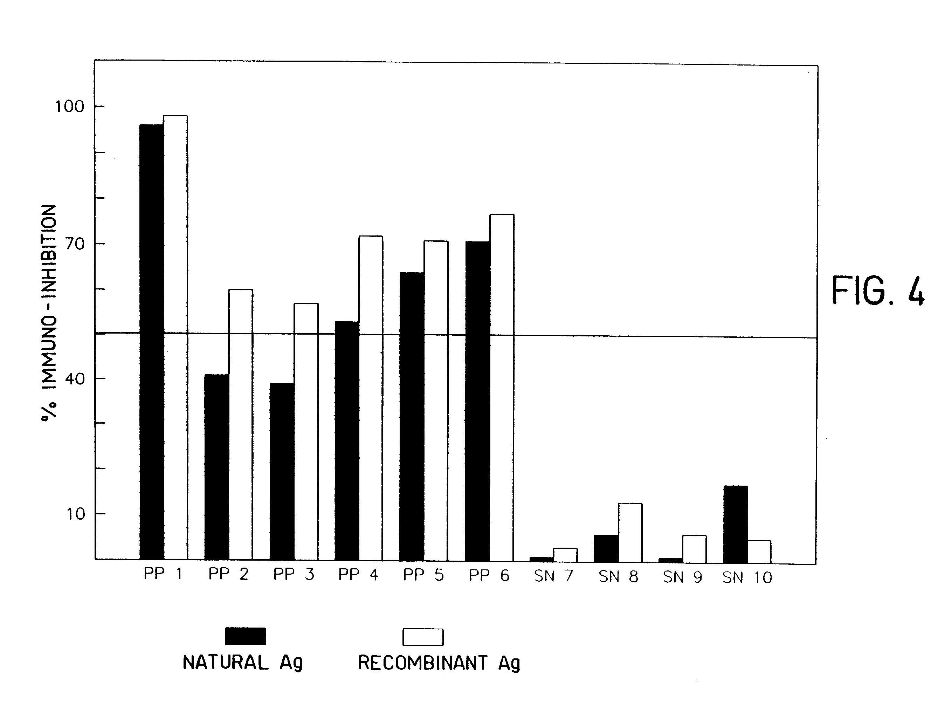

The r-HDAg purified as above is used in commercially available assays to define the total HDV antibodies (ETI-B-DELTAK and AB-DELTAK(RIA), SORIN Biomedica, Italy), for comparison purpose with the natural antigen from a lysate from HDV-infected marmot liver. Both antigens are used to analyse a series of anti-HDV immunoreactive serums (fig.4). The recombinant antigen (r-HDAg) has a comparable ability to recognise the HDV antibodies in the RIA assay as the natural source antigen; in the immunoenzymatic assay, r-HDAg is able to bind the HDV antibodies in PP2 and PP3 serums, that could not be detected by the natural antigen (fig.4).

### Example 3 Purification Of HCV r-P22 (Core Antigen) From S. frugiperda (Sf9) Insect Cells

### Lysis

Sf9 cells (ATCC CRL1711) are infected with pVL1393 baculum-type virus vector (Invitrogen, California, USA), wherein a 380 bp fragment coding for the first 120 aminoacids of the rp-22 protein (capsidic) of HCV virus is cloned in the EcoRI/PstI sites. The cells are cultivated by conventional methods.

The cell pellet (1 x 10⁹ cells) is resuspended in 15 ml TE (10 mM Tris-HCl; pH 8.0; 1 mM EDTA) and submitted to 100 W ultrasonic impulses for 1 minute; the procedure as above is repeated 4 times. The cell lysis is centrifuged at 100,000 x g for 60 minutes at 5°C. The pellet (membrane fraction) is resuspended in 10 ml of an A buffer. The solution is then stirred for 60 minutes at 5°C.

### Organic Precipitation

The suspension is added with 11 ml of B solution and stirred for 60 minutes at 5°C and then centrifuged at 100,000 x g at 10°C.

### Reverse Phase Chromatography

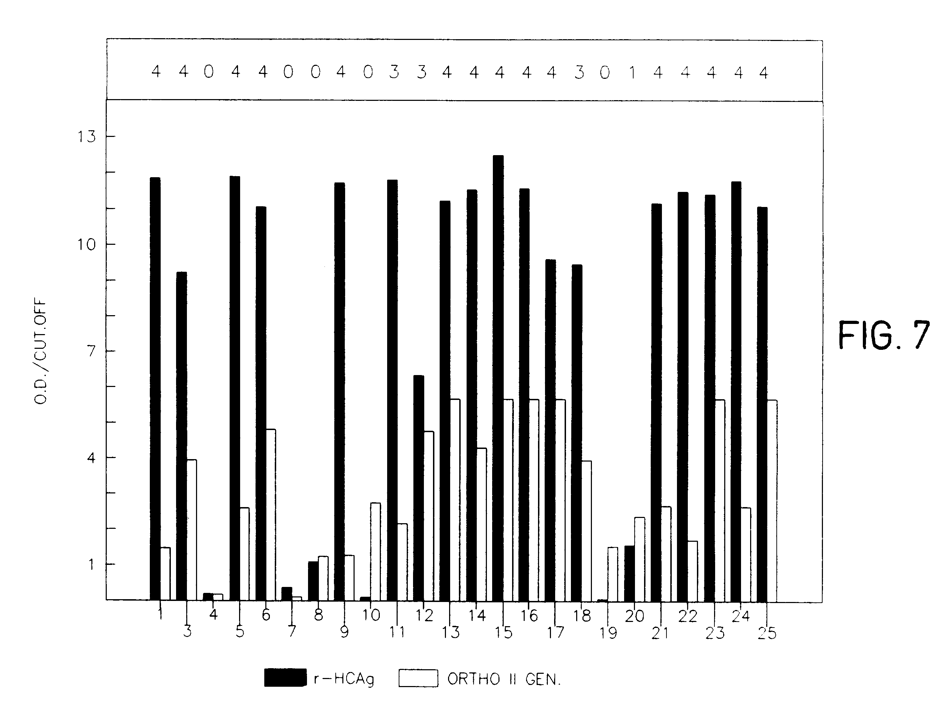

The supernatant (21 ml, fig.2) is loaded on a C4 reverse phase column (19 mm x 300 mm, Waters, C4 300 A), balanced with 25 % CH₃CN in 0.1 % TFA. The C4 column is eluted with a linear 25-30 % CH₃CN in 0.1 % TFA gradient (time: 30 minutes; flow rate: 10 ml/min.; detector: 280 nm); and as soon as the procedure is completed, all unadsorbed material is removed. The outlined peak in fig.6 shows r-p22 immunoreactivity. The r-p22 recombinant antigen (HCV core protein), after having been lyophilised and then resuspended in 2 % acetic acid, is analysed on a 15 % SDS PAGE , showing a purity level higher than 90 % (lane 3, fig.5).

### r-p22 Recombinant Antigen Immunoreactivity Check

The HCV antibody binding ability of the recombinant antigen is checked on a anti-HCV certified serum panel (Anti-HCV Mixed Titer Performance Panel (PHV201), Boston Biomedica, Inc., USA). The purified r-p22 protein, adsorbed on microtiter plates (Nunc, Danemark) is tested on a commercially available assay (ETI-AB-HCVK, SORIN Biomedica, Italy), by substituting the kit solid phase. The assay, modified as above, is compared with the commercially available ORTHO ELISA Second Generation (Ortho Diagnostic Systems, USA, fig.7). The ability of the r-p22 polypeptide in recognising HCV core protein antibodies is significant higher than the recognising ability of the used antigen in the Ortho Diagnostic System's assay (fig.7).

### LITERATURE

1. Mitraki, A. and King, J. "Biotechology", 690-697 (1989).
2. Bowdwn, G.A, Paredes, A.M. and Georgiu, G., "Biotecnology" 9, 725-730 (1991).
3. Haase-Pettingell, C.A and King. J., "J. of Biol. Chem.", 263, 4977-4983 (1988).
4. Hartley, D.L, and Kane, J.F., "Biochem. Soc. Trans", 16, 101-102 (1988).
5 . Frankel, S. ,Sohn, R. and Leinwand, L. "Proc . Natl . Acad. Sci. USA", 88, 1192-1196 (1991).
6. Hellebust , H ., Murby, M., Abramsen, L ., Uhlen M. and S-V Enfors "Biotechnology", 7, 165-168 (1989).
7 . Babbitt, P . C, West, B . L ., Buechter, D . D ., Kuntz, I.D. and Kenyon, G.L., "Biotechnology" 8, 945-949 (1990).
8 . Chang , M . F ., Baker , S . C ., Soe L . H ., Kamahora , T ., Keck , J . G ., Makino , S ., Gonvidara j an , S . and Lai , M.M.C., "J. Virol.", 62, 2403-2410, (1988).
9. Chao, M., Hsien, S.Y. and Taylor, J.M., "J. Virol.", C4, 5066-5069 (1990).
10. Kos, A., Molijn, A.C.M., Blauw, B. and Schellekens, H., "J. Gen. Virol. ", 72, 833-842, (1991) .
11. Xia, Y.P., Yeh, C.T., Ou J.H. and Lai M.M.C., "J. Virol.", 66, 914-921, (1992).

## Claims

1. A process to purify recombinant proteins having a basic isoelectric point from heterologous cell systems, comprising the steps of:
• processing cells, or an already purified fraction thereof, containing said proteins with basic isoelectric point, with a denaturing agent under acid environment conditions, in order to obtain a mixture;
• precipitating said mixture by using an organic solvent so to obtain a pellet and a supernatant;
• fractionating said supernatant by phase chromatography.

2. A process to purify recombinant proteins with basic isoelectric point according to claim 1, wherein said denaturing agent comprises a guanidine salt.

3. A process to purify recombinant proteins with basic isoelectric point according to claim 2, wherein said guanidine salt is guanidine hydrochloride.

4. A process to purify recombinant proteins with basic isoelectric point according to claim 3, wherein said guanidine hydrochloride is at a concentration comprised between 6 M and 8 M.

5. A process to purify recombinant proteins with basic isoelectric point according to claim 4, wherein said guanidine hydrochloride is at a concentration of 7 M.

6. A process to purify recombinant proteins with basic isoelectric point according to any of previous claim, wherein said organic solvent comprises acetonitrile in the presence of a volatile acid.

7. A process to purify recombinant proteins with basic isoelectric point according to claim 6, wherein said volatile acid is trifluoroacetic acid.

8. A process to purify recombinant proteins with basic isoelectric point according to claim 7, wherein the acetonitrile final concentration is in the range from 20 to 30 % v/v, and the trifluoroacetic acid concentration is in the range from 0.02 to 0.25% v/v.

9. A process to purify recombinant proteins with basic isoelectric point according to any of previous claim, wherein said phase chromatography comprises a chromatography on an inert silica resin.

10. A process to purify recombinant proteins with basic isoelectric point according to any previous claim, wherein said chromatography is a reverse phase chromatography and said protein is eluted with said organic solvent.

11. A process to purify recombinant proteins with basic isoelectric point according to any of previous claim, wherein said heterologous cell systems are prokaryote cells.

12. A process to purify recombinant proteins with basic isoelectric point according to any of previous claim, wherein said prokaryote cells are bacterial cells.

13. A process to purify recombinant proteins with basic isoelectric point according to claim 12, wherein said bacterial cells are *E. coli* cells.

14. A process to purify recombinant proteins with basic isoelectric point according to any of claims from 1 to 10, wherein said heterologous cell systems are eukaryote cells.

15. A process to purify recombinant proteins with basic isoelectric point according to claim 14, wherein said eukaryote cells are yeast, or insect or mammalian cells.

16. A process to purify recombinant proteins with basic isoelectric point according to claim 15, wherein said cells are *S. frugiperda* cells.

17. A process to purify recombinant proteins with basic isoelectric point according to any of previous claims, wherein said protein is r-HDAg of HDV.

18. A process to purify recombinant proteins with basic isoelectric point according to any of claims from 1 to 16, wherein said protein is r-p22 ("core"antigen) of HCV.

## Patentansprüche

1. Verfahren zur Reinigung von rekombinanten Proteinen mit basischem isoelektrischen Punkt aus Hetero-Zellsystemen, das folgende Vefahrensschritte hat:
- Behandlung von Zellen oder einer schon bereinigten Fraktion derselben, welche Proteine mit basischem isoelektrischen Punkt enthalten, durch ein Denaturierungsmittel unter säuren Umgebungsbedingungen, zur Erhaltung eines Gemisches;
- Ausfällung des vorgenannten Gemisches durch ein organisches Lösemittel zur Erhaltung einer Pille und eines Supernatants;
- Fraktionierung des vorgenanten Supernatants durch Phase-Chromatographie.

2. Verfahren zur Reinigung von rekombinanten Proteinen mit basischen isoelektrischen Punkt nach Anspruch 1, worin das vorgenannte Denaturisierungsmittel ein Guanidinsalz enthält.

3. Verfahren zur Reinigung von rekombinanten Proteinen mit basischen isoelektrischen Punkt nach Anspruch 2, worin das vorgenannte Guanidinsalz ein Guanidinchlorid ist.

4. Verfahren zur Reinigung von rekombinanten Proteinen mit basischen isoelektrischen Punkt nach Anspruch 3, worin das vorgenannte Guanidinchlorid zwischen 6M und 8M konzentriert ist.

5. Verfahren zur Reinigung von rekombinanten Proteinen mit basischen isoelektrischen Punkt nach Anspruch 3, worin das vorgenannte Guanidinchlorid bis auf 7M konzentriert ist.

6. Verfahren zur Reinigung von rekombinanten Proteinen mit basischen isoelektrischen Punkt nach einem der vorhergehenden Ansprüche, worin das vorgenannte Lösungsmittel Acetonitril in Anwesenheit einer flüchtigen Säure enthält.

7. Verfahren zur Reinigung von rekombinanten Proteinen mit basischen isoelektrischen Punkt nach Anspruch 6, worin die vorgenannte flüchtigen Säure eine Trifluoressigsäure ist.

8. Verfahren zur Reinigung von rekombinanten Proteinen mit basischen isoelektrischen Punkt nach Anspruch 7, worin das Acetonitril eine Endkonzentration im Bereich 20 bis 30 Vol.% hat und die Konzentration der Trifluoressigsäure im Bereich 0,02 bis 0,25 Vol.% enthalten ist.

9. Verfahren zur Reinigung von rekombinanten Proteinen mit basischen isoelektrischen Punkt nach einem der vorhergehenden Ansprüche, worin die vorgenannte Phasenchromatographie eine Chromatographie auf einem inerten Siliciumdioxid enthält.

10. Verfahren zur Reinigung von rekombinanten Proteinen mit basischen isoelektrischen Punkt nach einem der vorhergehenden Ansprüche, worin die vorgenannte Chromatographie eine phasenumgekehrte Chromatographie ist und das vorgenannte Protein mit dem vorgenannten organischen Lösemittel eluiert wird.

11. Verfahren zur Reinigung von rekombinanten Proteinen mit basischen isoelektrischen Punkt nach einem der vorhergehenden Ansprüche, worin das vorgenannten Hetero-Zellsysteme aus Prokaryoten-Zellen bestehen.

12. Verfahren zur Reinigung von rekombinanten Proteinen mit basischen isoelektrischen Punkt nach einem der vorhergehenden Ansprüche, worin die vorgenannten Prokaryoten-Zellen aus Bakterien-Zellen bestehen.

13. Verfahren zur Reinigung von rekombinanten Proteinen mit basischen isoelektrischen Punkt nach Anspruch 7, worin die vorgenannten Bakterien-Zellen aus *Escherichia Coli-Zellen* bestehen.

14. Verfahren zur Reinigung von rekombinanten Proteinen mit basischen isoelektrischen Punkt nach Ansprüche 1 bis 10, worin die vorgenannten Hetero-Zellen aus Eukaryote-Zelle bestehen.

15. Verfahren zur Reinigung von rekombinanten Proteinen mit basischen isoelektrischen Punkt nach Anspruch 14. wonn die vorgenannten Eukaryote-Zellen aus Hefe oder aus Insekten- oder Säugetierzellen bestehen.

16. Verfahren zur Reinigung von rekombinanten Proteinen mit basischen isoelektrischen Punkt nach Anspruch 15, worin die vorgenannten Zellen S. *Frugiperda-Zellen* sind.

17. Verfahren zur Reinigung von rekombinanten Proteinen mit basischen isoelektrischen Punkt nach einem der vorhergehenden Ansprüche, worin das vorgenannte Protein r-HDAg von HDV ist.

18. Verfahren zur Reinigung von rekombinanten Proteinen mit basischen isoelektrischen Punkt nach Ansprüche 1 bis 16, worin das vorgenannte Protein r-p 22 ("Kern" - Antigen) von HCV ist.

## Revendications

1. Procédé de purification de protéines recombinants ayant un point basique isoélectrique à partir de systèmes cellulaires, comprennant les phases de:
- traitement de celles ou d'une fraction purifiée des mêmmes, contenants lesdites protéines avec le point basique isoelectrique, au moyens d'un agent de dénaturation en conditions ambiant acides, pour obtenir un mélange;
- préecipitation dudit mélange en utilisand un solvant organique pour obtenir une pilule et un supernatant;
- fractionnement dudit supernatant par la chromatographie à phases.

2. Procédé de purification de protéines recombinants ayant un point basique isoélectrique selon la revendication 1, dans lequel ledit agent de dénaturation comprend un sel de guanidine.

3. Procédé de purification de protéines recombinants ayant un point basique isoélectrique selon la revendication 2, dans lequel ledit sel de guanidine est l'hydrochlorure de guanidine.

4. Procédé de purification de protéines recombinants ayant un point basique isoélectrique selon la revendication 3, dans lequel ledit hydrochlorure de guanidine a une concentration entre 6M et 8M.

5. Procédé de purification de protéines recombinants ayant un point basique isoélectrique selon la revendication 4, dans lequel ledit hydrochlorure de guanidine a une concentration de 7M.

6. Procédé de purification de protéines recombinants ayant un point basique isoélectrique selon l'une quelconque des revendications précédentes, dans lequel ledit solvant organique comprend acetonitrile en présence d'un acide volatile.

7. Procédé de purification de protéines recombinants ayant un point basique isoélectrique selon la revendication 6, dans lequel ledit acide volatile est un acide trifluoroacetique.

8. Procédé de purification de protéines recombinants ayant un point basique isoélectrique selon la revendication 7, dans lequel la concentration finale de l'acetonitrile est entre 20 et 30% Vol. et l'acide trifluoroacetique a une concentration entre 0,02 et 0,25% Vol.

9. Procédé de purification de protéines recombinants ayant un point basique isoélectrique selon des revendications précédentes, dans lequel la dite chromatographie à phases comprend une chromatographie sur un bioxyde de silicium.

10. Procédé de purification de protéines recombinants ayant un point basique isoélectrique selon des revendications précédentes, dans lequel ladite chromatographie est une chromatographie à phase inverse et ladite protéine est éluée par ledit solvant organique.

11. Procédé de purification de protéines recombinants ayant un point basique isoélectrique des revendications précédentes, dans lequel les systèmes hétérocellulaires sont des cellules procaryotes.

12. Procédé de purification de protéines recombinants ayant un point basique isoélectrique des revendications précédentes, dans lequel ledites cellules procaryotes sont des cellules bactérienes.

13. Procédé de purification de protéines recombinants ayant un point basique isoélectrique selon la revendication 12, dans lequel lesdites cellulas bactérienes sont des cellules *E. coli*.

14. Procédé de purification de protéines recombinants ayant un point basique isoélectrique selon les revendications 1 à 10, dans lequel lesdits systémes hétérocellulaires sont des cellules eucaryotes.

15. Procédé de purification de protéines recombinants ayant un point basique isoélectrique selon la revendication 14, dans lequel lesdites cellules eucaryotes sont des levains, ou des cellules de insects ou des cellules mammaires.

16. Procédé de purification de protéines recombinants ayant un point basique isoélectrique selon la revendication 15, dans lequel lesdites cellules sont des cellules S. *frugiperda.*

17. Procédé de purification de protéines recombinants ayant un point basique isoélectrique selon des précédentes, dans lequel ladite protéine est r-HDAg de HDV.

18. Procédé de purification de protéines recombinants ayant un point basique isoélectrique selon des précédents 1 à 16, dans lequel ladite protéine est r-p22 (antigène de "noyau") de HCV.
